# EUROPEAN PATENT SPECIFICATION

(11) **EP 1 131 100 B1**
(45) Date of publication and mention of the grant of the patent: **12.03.2003**
(21) Application number: 00903278.0
(22) Date of filing: 14.01.2000
(51) Int. Cl.: A61K 41/00, A61K 31/661, A61K 31/663, A61P 19/08

(54) **THERAPEUTIC COMPOSITIONS FOR METABOLIC BONE DISORDERS OR BONE METASTASES COMPRISING A PHOTOSENSITIZER AND A BISPHOSPHONATE**
THERAPEUTISCHE ZUSAMMENSETZUNGEN FUR KNOCHENSTOFFWECHSELSTÖRUNGEN ODER KNOCHENMETASTASEN ENTHALTEND EINEN PHOTOSENSITIZER UND EIN BISPHOSPHONAT
COMPOSITIONS AU TRAITEMENT DE TROUBLES METABOLIQUES OSSEUX ET DE METASTASES OSSEUSES CONTENANT UN AGENT PHOTOSENSITIVE ET UN BISPHOSPHONATE

(30) Priority: 15.01.1999 US 116233 P
(43) Date of publication of application: 12.09.2001
(73) Proprietor: Light Sciences Corporation, Issaquah, Washington 98027 (US)
(72) Inventor: CHEN, James, Bellevue, WA 98004 (US)
(74) Representative: Baldock, Sharon Claire
(86) International application number: US0000848
(87) International publication number: WO00041725

(56) References cited:
- US-A- 5 494 793
- US-A- 5 543 514
- US-A- 5 565 552
- DATABASE CHEMABS [Online] CHEMICAL ABSTRACTS SERVICE, COLUMBUS, OHIO, US; MEEROVICH, GENNADY A. ET AL: "Photosensitizer for PDT based on phosphonate phthalocyanine derivative" retrieved from STN Database accession no. 126:115157 XP002141100 & PROC. SPIE-INT. SOC. OPT. ENG. (1996), 2924(PHOTOCHEMOTHERAPY: PHOTODYNAMIC THERAPY AND OTHER MODALITIES II), 86-90 ,
- SHARMAN, WESLEY M. ET AL: "Novel water-soluble phthalocyanines substituted with phosphonate moieties on the benzo rings" TETRAHEDRON LETT. (1996), 37(33), 5831-5834 , XP002141099
- DATABASE BIOSIS [Online] BIOSCIENCES INFORMATION SERVICE, PHILADELPHIA, PA, US; NEMOTO, R. (1) ET AL: "Inhibition by a new bisphosphonate (YM175) of bone resorption induced by the MBT-2 tumour of mice." retrieved from STN XP002141101 & BRITISH JOURNAL OF CANCER, (1993) VOL. 67, NO. 5, PP. 893-897. ,

## Description

### TECHNICAL FIELD OF THE INVENTION

This invention relates generally to the field of medicine and pharmacotherapeutics with photosensitizing agents or other energy activated agents. Specifically, this invention relates to compounds, compositions and kits useful for targeting and treating metabolic bone disorders and bone metastases with photodynamic therapy (PDT).

### BACKGROUND OF THE INVENTION

A balanced physiological process of bone resorption, mediated by osteoclasts, and new bone formation, mediated by osteoblasts, maintains normal skeletal integrity. Enhanced bone resorption, however, is typical of metabolic bone disorders such as Paget's Disease, malignant hypercalcemia, osteoporosis and bone metastases. Paget's Disease is characterized by enhanced osteoclastic activity followed by abnormal osteoblast proliferation and increased bone formation. Hypercalcemia, a frequent complication of breast, prostate, lung and hematopoietic malignancies, may result from the direct lytic effect of tumor cells on bone, osteoclast activation by paracrine factors released from tumor cells, or increased renal calcium. Bone metastases are another frequent complication of breast, prostate, lung and hematopoietic malignancies. Osteoporosis is caused by either increased osteoclastic activity and accelerated bone resorption or reduced osteoclastic activity.

Adverse complications of metastatic bone disorders such as these include pain, pathologic fractures, spinal cord compression, hypercalcemia and immobility. Current therapies are palliative and include radiotherapy, radiopharmaceuticals, surgery, endocrine therapy, chemotherapy and bisphosphonates.

Bisphosphonates are synthetic analogs of naturally occurring inorganic pyrophosphates and have been used for many years in the treatment of Paget's Disease and hypercalcemia because like inorganic pyrophosphates, bisphosphonates bind to hydroxyapatite crystals in mineralized bone matrix, inhibit the recruitment and function of osteoclasts and stimulate osteoblasts to produce an inhibitor of osteoclast formation. (See: S. E. Papapoulos, *Medicina (BuenosAires) 57* (Suppl. I):61-64 (1997); and D. L. Lourwood, *Pharmacotherapy,* 18(4):779-789 (1998)) Bisphosphonates are resistant to metabolic and enzymatic inactivation by skeletal pyrophosphatases as they contain a phosphorous-carbon-phosphorous backbone rather than the phosphorous-oxygen-phosphorous backbone of pyrophosphates.

There are adverse side effects associated with bisphosphonate therapies such as nausea, vomiting, heartburn, diarrhea, gastrointestinal ulceration, osteomalacia, bone pain, increased fracturing, acute renal failure, hearing loss and toxic skin reactions are associated with the use of bisphosphonates. Generally, these adverse side effects are addressed by administering lower dosages, decreasing the frequency or periods of treatments and/or discontinuing therapy. Consequently, the lower dosages and decreased treatments decrease the efficacy of bisphosphonate therapy. Furthermore, although bisphosphonates are efficacious in treating bony metastatic disease, they are not anticancer agents per se. Therefore, bisphosphonates alone are not effective in treating cancer, and rather are used to palliate symptoms such as pain.

Although, photodynamic therapy (PDT) has received increasing interest as a mode of treatment for a wide variety of different cancers, PDT for the treatment of metastatic bone disease is underdeveloped. Furthermore, PDT is often associated with inadvertent tissue damage normal tissue adjacent to diseased tissue to be treated. This inadvertent damage to collateral tissues is due to the nonspecific uptake of the photosensitizer by tissue the photosensitizer perfuses. Thus, a non-specific uptake of photosensitizer by bone tissue during PDT could potentially damage normal bone tissue that has been replaced by the abnormal bone formation associated with a particular disorder such as Paget's Disease.

Clearly, the acknowledged side effects and probable lack of efficacy of the current PDT therapies against skeletal metastases present a need for a different approach for the treatment of metastatic bone diseases.

Citation of the above documents is not intended as an admission that any of the foregoing is pertinent prior art. All statements as to the date or representation as to the contents of these documents is based on the information available to the applicants and does not constitute any admission as to the correctness of the dates or contents of these documents.

### SUMMARY OF THE INVENTION

The present disclosure teaches compositions and the use of said compositions in the manufacture of a medicament for treating metabolic bone disorders and bone metastases.

The present invention relates to bisphosphonate conjugates or pyrophosphate conjugates and their administration for PDT treatment of metabolic bone disorders and metastases.

Specifically, the present invention relates to the use of said compositions in the manufacture of a medicament for the treatment of metabolic bone disorders and metastases by the precise targeting of photosensitive agents or other energy activated agents, drugs and compounds to the target pathologic bone cells or pathologic bone tissues or skeletal metastases due to cancer of a mammalian subject, and activating these targeted photosensitizers by subsequently administering to the subject light or ultrasonic energy of a relatively low fluence rate over a prolonged period of time from a light or ultrasonic energy source that is either external or internal to the target tissues in order to achieve maximal cytotoxicity with minimal side effects.

One embodiment of the present invention is drawn to compositions comprising photosensitive agents conjugated to a compound selected from the group consisting of: bisphosphonates; pyrophosphonates; thiobisphosphonates; and nitrobisphosphonates. The photosensitizing agent is selected from the group consisting of: indocyanine green (ICG); methylene blue; toluidine blue; aminolevulinic acid (ALA); chlorin compounds; phthalocyanines; porphyrins; purpurins; texaphyrins; and any other agent that absorbs light in a range of 500 nm-1100 nm. A preferred embodiment of this invention contemplates that the photosensitizing agent is indocyanine green (ICG) and the compound conjugated to ICG is a bisphosphonate. These conjugates may be further conjugated to another ligand where the ligand is a target tissue specific antibody, peptide or polymer.

Another embodiment of the resent invention is drawn to the use of these bisphosphonate compositions in the manufacture of a medicament for PDT treatment of diseased tissues related metabolic bone disorders and bone metastases due to cancer. These uses generally comprise: administering to the subject a therapeutically effective amount of a bisphosphonate composition, where the bisphosphonate composition selectively binds to the pathologic target tissue. This step is followed by irradiating at least a portion of the subject with light at a wavelength or waveband absorbed by the bisphosphonate composition, where the light is provided by a light source, and where the irradiation is at a relatively low fluence rate that results in the activation of the bisphosphonate composition. In this embodiment of the present invention, the bisphosphonate composition is cleared from non-target tissues of the subject prior to irradiation.

A further embodiment of the present invention is drawn to the use as described above, and includes the steps of imaging the target tissue and determining the sites of irradiation.

Another embodiment of the present invention is drawn to the use in a method of PDT of a target tissue in a mammalian subject as described above, where the light source is external to the patient's intact skin layer. A further embodiment of this invention is drawn to this method of PDT wherein the light source is inserted underneath the patient's intact skin layer, but is external to an intact organ surface, where the organ comprises the target tissue. Still a further embodiment of the present invention of PDT contemplates that the light source is inserted underneath the patient's intact skin layer and is inserted into an organ, where the organ comprises the target tissue.

Another preferred embodiment contemplates the use in a transcutaneous PDT method where the photosensitizing agent delivery system comprises a liposome delivery system consisting essentially of bisphosphonate compositions.

### BRIEF DESCRIPTION OF THE DRAWINGS

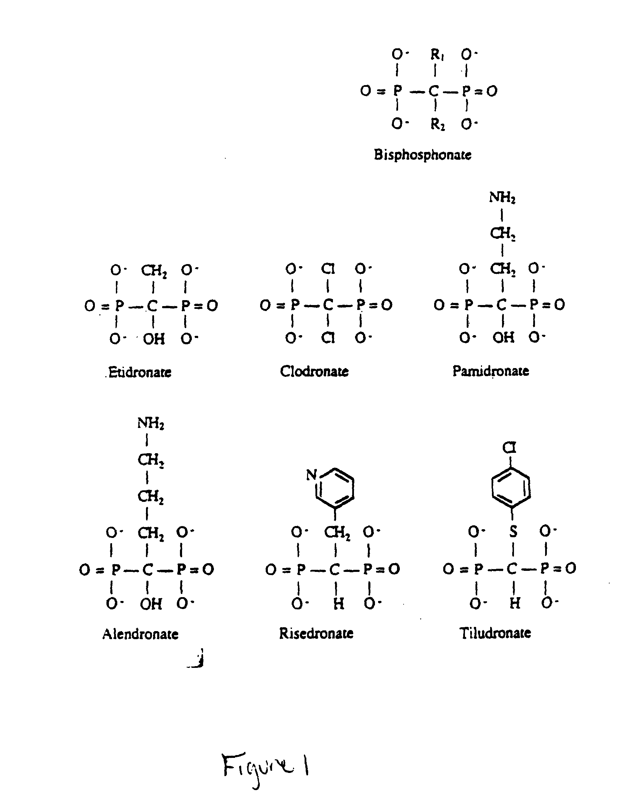

Figure 1 shows the structure of bisphosphonates; including: etidronate; pamidronate; risedronate; clodronate; alendronate; ibandronate and tiludronate.

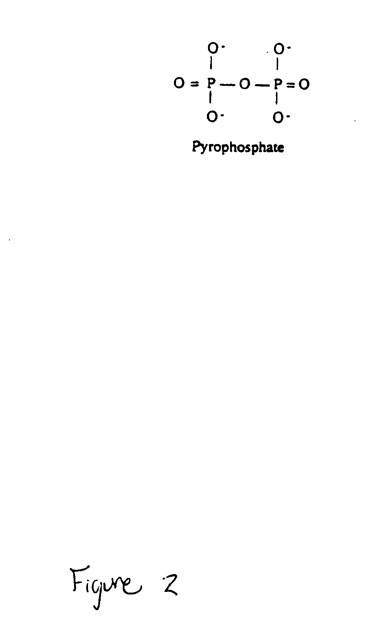

Figure 2 shows the structure of pyrophosphonate.

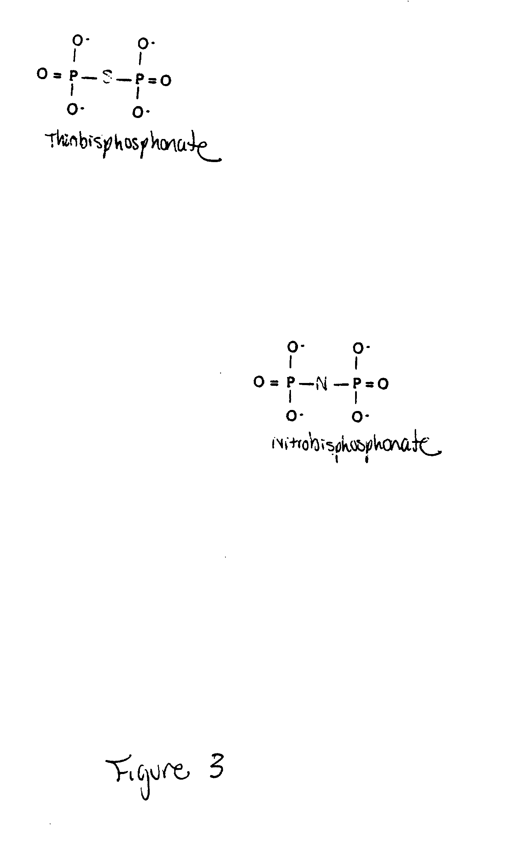

Figure 3 shows the structure of nitrobisphosphonates and thiobisphosphonates.

### DETAILED DESCRIPTION OF THE INVENTION

This invention provides compositions and the use of said compositions in the manufacture of a medicament for treating diseased tissues related to metabolic bone disorders and metastases in mammalian subjects. The compositions are bisphosphonates, pyrophosphates or bisphosphonate-like compounds conjugated to photosensitive agents which are optionally further conjugated to ligands which are target tissue specific antibodies, peptides or polymers.

These compositions are used in the manufacture of a medicament for PDT treatment to target the tissues or cells of a mammalian subject to be treated. The uses comprise irradiating at least a portion of the subject with light at a wavelength or waveband absorbed by said photosensitizing agent that under conditions of activation during photodynamic therapy using a relatively low fluence rate, but an overall high total fluence dose resulting in minimal collateral normal tissue damage.

Generally, PDT is performed by first administering a photosensitive compound systemically or topically, followed by illumination of the treatment site at a wavelength or waveband which closely matches the absorption spectra of the photosensitizer. In doing so, singlet oxygen and other reactive species are generated leading to a number of biological effects resulting in cytotoxicity. The depth and volume of the cytotoxic effect in tissue depends on the complex interactions of light penetration in tissue, the photosensitizer concentration and cellular location, and availability of molecular oxygen.

Terms as used herein are based upon their art recognized meaning and from the present disclosure should be clearly understood by the ordinary skilled artisan. For sake of clarity, terms may also have particular meaning as would be clear from their use in context. For example, transcutaneous more specifically herein refers to the passage of light through unbroken tissue. Where the tissue layer is skin or dermis, transcutaneous includes transdermal and the light source is external to the outer skin layer. Transillumination refers herein to the passage of light through a tissue layer, such as the outer ocortex layer of an organ such as bone, where the light source is external to the organ, but internal or implanted into the subject or patient.

Specifically, the present invention is based on the precise targeting of photosensitive agents or drugs and compounds to specific target antigens of a subject or patient and to the method of activation of targeted photosensitizer agents by subsequently administering to the subject light of a relatively low fluence rate over a prolonged period of time from a light source that is external to the target tissue in order to achieve maximal cytotoxicity with minimal side effects or collateral tissue damage.

Further, as used herein "target cells" or "target tissues" are those cells or tissues, respectively that are intended to be impaired or destroyed by this treatment method. Target cells or target tissues take up the photosensitizing agent; then when sufficient radiation is applied, these cells or tissues are impaired or destroyed. Target cells are those cells in target tissues related to those involved in metabolic bone disorders and bone metastases. Also included among target cells are cells undergoing rapid division as compared to non-target cells. The term "target cells" also includes, but is not limited to, microorganisms such as bacteria, viruses, fungi, parasites and other infectious agents which may be infecting a bony tissue. Thus, the term "target cell" is not limited to living cells but also includes infectious particles such as viruses.

"Non-target cells" are all the cells of an intact animal which are not intended to be impaired or destroyed by the treatment method. These non-target cells include but are not limited to healthy bone cells, and other normal bone tissue, not otherwise identified to be targeted.

"Destroy" is used to mean kill the desired target cell. "Impair" means to change the target cell in such a way as to interfere with its function. "Photosensitive agent" is a chemical compound which when contacted by radiation, absorbs the light, which results in impairment or destruction of the target cells. Virtually any chemical compound that homes to a selected target and absorbs light may be used in this invention. Preferably, the chemical compound is nontoxic to the animal to which it is administered or is capable of being formulated in a nontoxic composition. Preferably, the chemical compound in its photodegraded form is also nontoxic. A comprehensive listing of photosensitive chemicals may be found in Kreimer-Birnbaum, Sem. Hematol. 26:157-73, 1989.

Photosensitive compounds include, but are not limited to, chlorins, bacteriochlorins, phthalocyanines, porphyrins, purpurins, merocyanines, psoralens, benzoporphyrin derivatives (BPD) and porfimer sodium and pro-drugs such as delta-aminolevulinic acid, which can produce drugs such as protoporphyrin. Other compounds include indocyanine green (ICG); methylene blue; toluidine blue; texaphyrins; and any other agent that absorbs light in a range of 500 nm -1100 nm.

Bisphosphonates, pyrophosphates and bisphosphonate-like compounds, collectively referred to herein as "bisphosphonates" are those compounds exhibiting the characteristics of compounds having a phosphate-oxygen-phosphate or phosphate-carbon-phosphate backbone which characteristics comprise the ability to bind strongly calcium crystals and affect osteoclast-mediated bone resorption. Examples of such bisphosphonates are, but are not limited to, etidronate, tiludronate, clodronate, pamidronate, alendronate, risedronate and ibandronate.

"Bisphosphonate compositions" are photosensitive agents conjugated to bisphosphonates, pyrophosphates, nitrobisphosphonates, thiobisphosphonates or other compounds having similar bisphosphonate-like properties and that also possess either an oxygen or carbon or nitrogen or sulfur atom bound to two phosphonate groups.

"Nitrobisphosphonates" are compounds comprising a nitrogen atom bound to two phosphonate groups.

"Thiobisphosphonates" are compounds comprising a sulfur atom bound to two phosphonate groups.

"Radiation" as used herein includes all wavelengths. Preferably, the radiation wavelength is selected to match the wave length(s) or wavebands which excites the photosensitive compound. Even more preferably, the radiation wavelength matches the excitation wavelength of the photosensitive compound and has low absorption by the non-target cells and the rest of the intact animal, including blood proteins. For example, the preferred wavelength for ICG is the range of 750-850 nm.

The radiation is further defined in this invention by its intensity, duration, and timing with respect to dosing with the photosensitive agent. The intensity or fluence rate must be sufficient for the radiation to penetrate skin and reach the target cells, target tissues or target compositions. The total fluence dose must be sufficient to photoactivate enough photosensitive agent to act on the target cells. Both intensity and duration must be limited to avoid overtreating the animal. Timing with respect to dosing with the photosensitive agent is important, because 1) the administered photosensitive agent requires some time to home in on target cells and 2) the blood level of many photosensitive agents decreases rapidly with time.

This invention provides a use of the claimed compositions in the manufacture of a medicament for treating an animal, which includes, but is not limited to, humans and other mammals. The term "mammals" or "mammalian subject" also includes farm animals, such as cows, hogs and sheep, as well as pet or sport animals such as horses, dogs and cats.

By "intact animal" is meant that the whole, undivided animal is available to be exposed to radiation. No part of the animal is removed for separate radiation, in contrast with photophoresis, in which the animal's blood is circulated outside its body for exposure to radiation. The entire animal need not be exposed to radiation. Only a portion of the intact animal subject may or need be exposed to radiation.

"Transcutaneously" is used herein as meaning through the skin of an animal subject.

Briefly, a bisphosphonate composition incorporating a photosensitizing agent is generally administered to the animal before the animal is subjected to radiation.

Preferred photosensitizing agents include, but are not limited to, chlorins, bacteriochlorins, phthalocyanines, porphyrins, purpurins, merocyanines, psoralens and pro-drugs such as .delta.-aminolevulinic acid, which can produce drugs such as protoporphyrin. More preferred are: methylene blue; toluidine blue; texaphyrins; and any other agent that absorbs light in a range of 500 nm -1100 nm. Most preferred is indocyanine green (ICG) (for example, see: WO 92/00106 (Raven *et al*.); WO97/31582 (Abels *et al.*) and Devoisselle *et al*., *SPIE* 2627:100-108, 1995).

The bisphosphonate composition is administered locally or systemically. The bisphosphonate composition is administered orally or by injection which may be intravenous, subcutaneous, intramuscular or intraperitoneal. The photosensitizing agent and/or a bisphosphonate composition also can be administered enterally or topically via patches or implants.

The bisphosphonate composition also can be conjugated to specific ligands reactive with a target, such as receptor-specific ligands or immunoglobulins or immunospecific portions of immunoglobulins, permitting them to be more concentrated in a desired target cell or microorganism. The photosensitizing agent and/or a bisphosphonate composition may be further conjugated to a ligand-receptor binding pair, which includes, but is not limited to: biotin-streptavidin; chemokine-chemokine receptor; growth factor-growth factor receptor; and antigen-antibody. This conjugation may permit lowering of the required dose level since the material is more selectively target and less is wasted in distribution into other tissues whose destruction must be avoided.

The bisphosphonate composition may also be conjugated to "imaging agents" such as technetium, radium, indium or gallium.

The bisphosphonate composition can be administered in a dry formulation, such as pills, capsules, suppositories or patches. The biphosphonate composition also may be administered in a liquid formulation, either alone with water, or with pharmaceutically acceptable excipients, such as are disclosed in Remington's Pharmaceutical Sciences. The liquid formulation also can be a suspension or an emulsion. Liposomal or lipophilic formulations may be desirable. If suspensions or emulsions are utilized, suitable excipients include water, saline, dextrose, glycerol, and the like. These compositions may contain minor amounts of nontoxic auxiliary substances such as wetting or emulsifying agents, antioxidants, pH buffering agents, and the like.

The dose of bisphosphonate composition will vary with the target cell(s) sought, the optimal blood level (see Example 1), the animal's weight and the timing of the irradiation. Depending on the bisphosphonate composition used, an equivalent optimal therapeutic level will have to be established. Preferably, the dose is calculated to obtain a blood level between about 0.001 and 100 µg/ml. Preferably, the dose will obtain a blood level between about 0.01 and 10 µg/ml.

The methods comprise irradiating at least a portion of the subject with light at a wavelength or waveband absorbed by said photosensitizing agent that under conditions of activation during photodynamic therapy using a relatively low fluence rate, but an overall high total fluence dose resulting in minimal collateral normal tissue damage. What is meant by "relatively low fluence rate" is a fluence rate that is lower than that typically used and one that generally does not result in significant damage to collateral or non-target tissues. Specifically, the intensity of radiation used to treat the target cell or target tissue is preferably between about 5 and 100 mW/cm.². More preferably, the intensity of radiation is between about 10 and 75 mW/cm.². Most preferably, the intensity of radiation is between about 15 and 50 mW/cm.².

The duration of radiation exposure is preferably between about 30 minute and 72 hours. More preferably, the duration of radiation exposure is between about 60 minutes and 48 hours. Most preferably, the duration of radiation exposure is between about 2 hours and 24 hours.

While not wishing to be limited by a theory, the inventor proposes that a photosensitizer agent can be substantially and selectively photoactivated in the target cells and target tissues within a therapeutically reasonable period of time and without excess toxicity or collateral damage to non-target tissues. Thus, there appears to be a therapeutic window bounded by the photosensitizer agent dosage and radiation dosage. The formation of photodegradation products of a photosensitizer agent was used as an indicator of photoactivation. Photoactivation of a photosensitizer agent has been postulated to cause the formation of singlet oxygen, which has a cytotoxic effect. In view of the problems related to current methods of treating skeletal metastases which are palliative, the envisaged method of targeted transcutaneous PDT of patients injected with a biphosphonate composition and subjected to a relatively low fluence rate, but high total fluence dose of irradiation is an attractive approach to the treatment of target tissues, that include neoplastic disease and infectious agents.

Additionally, the present invention is drawn to a use of the claimed compositions in the manufacture of a medicament for transcutaneous therapy of skeletal metastases in a mammalian subject or patient by first administering to the subject a therapeutically effective amount of a first conjugate comprising a first member of a ligand-receptor binding pair conjugated to an antibody or antibody fragment, wherein said antibody or antibody fragment selectively binds to a target tissue antigen; and simultaneously or subsequently administering to the subject a therapeutically effective amount of a second conjugate comprising a second member of the ligand-receptor binding pair conjugated to an biphosphonate composition or biphosphonate agent delivery system wherein the first member binds to the second member of the ligand-receptor binding pair. These steps are followed by irradiating or sonicating at least a portion of the subject with energy at a wavelength, waveband, or frequency absorbed by said biphosphonate composition or biphosphonate agent delivery system, by the product thereof, wherein said energy is provided by an energy source that is external to the subject; and wherein said light irradiation or sonication is at a low dose rate that results in the activation of said biphosphonate composition or biphosphonate agent delivery system

While the preferred embodiment of the present invention is drawn to the use of light energy in a photodynamic therapy of skeletal tumors other forms of energy are within the scope of this invention and understandable by one of ordinary skill in the art. Such forms of energy include, but are not limited to: thermal; ultrasonic; ultrasonic; chemical; photo or light; microwave; ionizing, such as: x-ray, and gamma ray;; and electrical. For example, sonodynamically induced or activated biphosphonate compositions include, but are not limited to: gallium-porphyrin complex (see: Yumita *et al., Cancer Letters,* 112: 79-86, 1997); other porphyrin complexes, such as protoporphyrin and hematoporphyrin (see: Umemura *et al., Ultrasonics Sonochemistry 3:* S187-S191, 1996); other cancer drugs, such as daunorubicin and adriamycin, used in the presence of ultrasound therapy (see: Yumita *et al., Japan J. Hyperthermic Oncology,* 3(2): 175-182, 1987).

This invention further contemplates the use of an energy source, preferably a light source, that is external to the target tissue. The target tissues may include and may relate to cells and tissues involved in metabolic bone disorders and metastases, per se.

The ordinary skilled artisan would be familiar with various ligand-receptor binding pairs, including those known and those currently yet to be discovered. Those known, include, but are not limited to the group consisting of: biotin-streptavidin; chemokine-chemokine receptor; growth factor-growth factor receptor; and antigen-antibody. This invention contemplates a preferred embodiment that includes the use of biotin-streptavidin as the ligand-receptor binding pair. However, the ordinary skilled artisan would readily understand from the present disclosure that any ligand-receptor binding pair may be useful provided the ligand-receptor binding pair demonstrate a specificity for the binding by the ligand to the receptor and further provided that the ligand-receptor binding pair permit the creation of a first conjugate comprising a first member of the ligand-receptor binding pair conjugated to an antibody or antibody fragment, wherein said antibody or antibody fragment selectively binds to a target tissue antigen; and further permit the creation of a second biphosphonate conjugate comprising a second member of the ligand-receptor binding pair conjugated to a photosensitizing agent or ultrasound sensitive agent, and further wherein the first member binds to the second member of the ligand-receptor binding pair.

A preferred embodiment of the present invention is drawn to a method where the photosensitizing agent delivery system includes a liposome delivery system consisting essentially of the bisphosphonate composition A still further and preferred embodiment of the present invention contemplates the disclosed method where the photosensitizing agent delivery system utilizes both a liposome delivery system and a bisphosphonate composition, where each is separately conjugated to a second member of the ligand-receptor binding pair, and where the first member binds to the second member of the ligand-receptor binding pair, and more preferably where the ligand-receptor binding pair is biotin-streptavidin. This embodiment further contemplates that the bisphosphonate composition as well as the photosensitizing agent delivery system may both be specifically targeted through the selective binding to a target tissue antigen by the antibody or antibody fragment of the first member binding pair. Such dual targeting is envisioned to enhance the specificity of uptake and to increase the quantity of uptake. Though the total fluence delivered to the treatment site will be variable depending on the size and nature of the treatment site, it is contemplated that the preferred total fluence delivered either internally or from an external light source will range between 30 Joules to 25,000 Joules, more preferably between 100 Joules to 20,000 Joules, and most preferably between 500 Joules to 10,000 Joules.

Having now generally described the invention, the same will be more readily understood through reference to the following examples which are provided by way of illustration, and are not intended to be limiting of the present invention, unless specified.

### EXAMPLE 1

### Photodynamic Therapy of Treating or Preventing Bone Metastases

A. A patient having or susceptible to bone metastases is given an oral or intravenous dose of a photosensitizer agent, indocyanine green (ICG), conjugated to a bisphosphonate which specifically binds the target tissue. One or more light sources are strategically placed or implanted near the tissue to be treated. Following a sufficient amount of time to permit clearing of the bisphosphonate composition from the non-target tissues, the light sources are activated, irradiating the target tissue with a relatively low fluence rate, but high total fluence dose of light in the wavelength from about 750 nm to about 850 nm. The light may be applied internally or from an external allocation, with the light effectively penetrating the skin and intervening tissue due to its long wavelength.
   The specific dose of biphosphonate conjugate is that which results in a concentration of active ICG sufficient to obtain a blood level between about 0.001 and 100 µg/ml. and more preferably, a dose of between about 0.01 and 10 µg/ml. However, it is well within the skill of the ordinary skilled artisan to determine the specific therapeutically effective dose using standard clinical practices and procedures.
   Additionally, as renal clearance is the only route of bisphosphonate elimination, and the amount of bisphosphonates not absorbed into bone tissue is excreted unchanged in urine, the specific therapeutically effective dose may be customized for an individual subject undergoing treatment by monitoring the urine levels of bisphosphonates.
   Similarly, the specific fluence rate and total fluence dose may be routinely determined from the disclosure herein.
   Furthermore, as most urinary excretion of bisphosphonates occurs within 12 hours of administration and little additional drug is recovered in the urine after 24 hours, the sufficient amount of time to permit clearing of the bisphosphonate composition from the non-target tissues preferred is 12-15 hours after administration of the bisphosphonate composition.
B. Alternatively, the bisphosphonate composition above could be further conjugated to an imaging agent such as technetium. Thus, the method as disclosed in A above could further comprise the steps of performing a nuclear medicine scan and imaging the metastatic sites to be treated.
C. The method as disclosed in A could further comprise the steps of administering a composition comprising bisphosphonate conjugated to an imaging agent such as technetium, performing a nuclear medicine scan and imaging the metastatic sites to be treated.

### EXAMPLE 2

### Photodynamic Therapy of Paget's Disease

As Paget's Disease is characterized by localized enhancement of osteoclastic activity and greater numbers of large osteoclasts, this disorder may be treated effectively with the PDT methods as described above. For example, a mammalian subject suffering from Paget's Disease is given an oral or intravenous dose of a photosensitizer agent, such as indocyanine green (ICG), conjugated to a bisphosphonate which selectively localizes to the sites osteoclastic activity. The bisphosphonate composition is further conjugated to an imaging agent, such as technetium, or another bisphosphonate composition conjugated to an imaging agent is also administered to the subject. Following a sufficient amount of time to permit clearing of the bisphosphonate composition from the non-target tissues, a nuclear medicine scan is then performed in order to determine the sites of abnormal osteoclastic activity and target the great numbers of large osteoclasts.

Then one or more light sources are strategically placed or implanted near the tissue to be treated, the light sources are activated, irradiating the target tissue with a relatively low fluence rate, but high total fluence dose of light in the wavelength from about 750 nm to about 850 nm. The light may be applied internally or externally.

The specific dose of photosensitizer conjugate is that which results in a concentration of active ICG sufficient to obtain a blood level between about 0.001 and 100 µg/ml. and more preferably, a dose of between about 0.01 and 10 µg/ml. However, it is well within the skill of the ordinary skilled artisan to determine the specific therapeutically effective dose using standard clinical practices and procedures.

Additionally, as renal clearance is the only route of bisphosphonate elimination, and the amount of bisphosphonates not absorbed into bone tissue is excreted unchanged in urine, the specific therapeutically effective dose may be customized for an individual subject undergoing treatment by monitoring the urine levels of bisphosphonates.

Similarly, the specific fluence rate and total fluence dose may be routinely determined from the disclosure herein.

Furthermore, as most urinary excretion of bisphosphonates occurs within 12 hours of administration and little additional drug is recovered in the urine after 24 hours, the sufficient amount of time to permit clearing of the bisphosphonate composition from the non-target tissues preferred is 12-15 hours after administration of the bisphosphonate composition.

As Paget's Disease is characterized by abnormally localized enhanced osteoclastic activity followed by abnormal bone formation of poor structural quality, this type of PDT treatment should minimize the bone pain, skeletal deformity, fractures, secondary arthritis, neurologic impairment and hearing loss. Since increased bone turnover is associated with increased serum levels of alkaline phosphatase and increased urinary excretion of hydrozyproline, deoxypyridinoline and cross-linked *N*-telopeptide of type I collagen, the efficacy of the treatment may be determined by the serum levels of alkaline phosphatase and/or the urine levels of hydrozyproline, deoxypyridinoline and cross-linked N-telopeptide of type I collagen. Usually, the success of the treatment is estimated by evaluating whether serum alkaline phosphatase has been reduced by 60% or lowered into the normal ranges.

### EXAMPLE 3

### Photodynamic Therapy of Hypercalcemia

If hypercalcemia in a subject results from the direct or indirect effect of tumor cells on bone resorption, PDT treatment may be effective in returning serum calcium levels to normal and reducing bone pain. For example, a mammalian subject suffering from hypercalcemia is given an oral or intravenous dose of a photosensitizer agent, such as indocyanine green (ICG), conjugated to a bisphosphonate which selectively localizes to the tumor cells on the bone and/or the osteoclasts activated by tumor cells. The bisphosphonate composition is further conjugated to an imaging agent, such as technetium, or another bisphosphonate composition conjugated to an imaging agent is also administered to the subject. Following a sufficient amount of time to permit clearing of the bisphosphonate composition from the non-target tissues, a nuclear medicine scan is then performed in order to determine the sites of the tumor cells on the bone and/or the osteoclasts activated by tumor cells.

Then one or more light sources are strategically placed or implanted near the tissue to be treated, the light sources are activated, irradiating the target tissue with a relatively low fluence rate, but high total fluence dose of light in the wavelength from about 750 nm to about 850 nm. The light may be applied internally or externally.

The specific dose of photosensitizer conjugate is that which results in a concentration of active ICG sufficient to obtain a blood level between about 0.001 and 100 µg/ml. and more preferably, a dose of between about 0.01 and 10 µg/ml. However, it is well within the skill of the ordinary skilled artisan to determine the specific therapeutically effective dose using standard clinical practices and procedures.

Additionally, as renal clearance is the only route of bisphosphonate elimination, and the amount of bisphosphonates not absorbed into bone tissue is excreted unchanged in urine, the specific therapeutically effective dose may be customized for an individual subject undergoing treatment by monitoring the urine levels of bisphosphonates.

Similarly, the specific fluence rate and total fluence dose may be routinely determined from the disclosure herein.

Furthermore, as most urinary excretion of bisphosphonates occurs within 12 hours of administration and little additional drug is recovered in the urine after 24 hours, the sufficient amount of time to permit clearing of the bisphosphonate composition from the non-target tissues preferred is 12-15 hours after administration of the bisphosphonate composition.

As hypercalcemia is characterized by abnormally high serum calcium levels, this type of PDT treatment should minimize the associated complications of hypercalcemia such as intense pain, pathologic fractures, changes in normal neurologic and cardiac function, coma, arrhythmias and death. Additionally, the success of the treatment may be evaluated by the amount of serum calcium levels.

### EXAMPLE 4

### Photodynamic Therapy of Type I Osteoporosis

As Type I osteoporosis is characterized by increased osteoclastic activity followed by accelerated bone resorption, this disorder may be treated effectively with the PDT methods as described above. For example, a mammalian subject suffering from Type I osteoporosis is given an oral or intravenous dose of a photosensitizer agent, such as indocyanine green (ICG), conjugated to a bisphosphonate which selectively localizes to the sites osteoclastic activity. One or more light sources are strategically placed or implanted near the tissue to be treated. Following a sufficient amount of time to permit clearing of the bisphosphonate composition from the non-target tissues, the light sources are activated, irradiating the target tissue with a relatively low fluence rate, but high total fluence dose of light in the wavelength from about 750 nm to about 850 nm. The light may be applied internally or externally.

The specific dose of photosensitizer conjugate is that which results in a concentration of active ICG sufficient to obtain a blood level between about 0.01 and 100 µg/ml. and more preferably, a dose of between about 0.01 and 10 µg/ml. However, it is well within the skill of the ordinary skilled artisan to determine the specific therapeutically effective dose using standard clinical practices and procedures.

Additionally, as renal clearance is the only route of bisphosphonate elimination, and the amount of bisphosphonates not absorbed into bone tissue is excreted unchanged in urine, the specific therapeutically effective dose may be customized for an individual subject undergoing treatment by monitoring the urine levels of bisphosphonates.

Similarly, the specific fluence rate and total fluence dose may be routinely determined from the disclosure herein.

Furthermore, as most urinary excretion of bisphosphonates occurs within 12 hours of administration and little additional drug is recovered in the urine after 24 hours, the sufficient amount of time to permit clearing of the bisphosphonate composition from the non-target tissues preferred is 12-15 hours after administration of the bisphosphonate composition.

Since increased bone turnover is associated with increased serum levels of alkaline phosphatase and increased urinary excretion of hydrozyproline, deoxypyridinoline and cross-linked *N*-telopeptide of type I collagen, the efficacy of the treatment may be determined by the serum levels of alkaline phosphatase and/or the urine levels of hydrozyproline, deoxypyridinoline and cross-linked *N*-telopeptide of type I collagen. Usually, the success of the treatment is estimated by evaluating whether serum alkaline phosphatase has been reduced by 60% or lowered into the normal ranges.

This invention has been described by a direct description and by examples. As noted above, the examples are meant to be only examples and not to limit the invention in any meaningful way. Additionally, one having ordinary skill in the art to which this invention pertains in reviewing the specification and claims which follow would appreciate that there are equivalents to those claimed aspects of the invention. The inventors intend to encompass those equivalents within the reasonable scope of the claimed invention.

## Claims

1. A pharmaceutical composition comprising a photosensitizer agent conjugated to a compound selected from the group consisting of: bisphosphonates; pyrophosphonates; thiobisphosphonates; and nitrobisphosphonates.

2. The composition of claim 1 wherein the photosensitizer agent is selected from the group consisting of chlorins, bacteriochlorins, phthalocyanines, porphyrins, purpurins, merocyanines, psoralens, benzoporphyrin derivatives (BPD), porfimer sodium, delta-aminolevulinic acid, protoporphyrin, indocyanine green (ICG), methylene blue, toluidine blue, texaphyrins and any other agent that absorbs light in a range of 500 nm -1100 nm.

3. The composition of claim 1 wherein the compound is a bisphosphonate of the formula 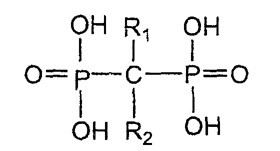 wherein R¹ is independently selected from the group consisting of: hydroxyl, an amino group, -CN, -NO₂, haloalkyl, heteroaryl, phenyl, alkyl, alkoxy, alkylthio, halo and alkyl-carbonyloxy; and wherein R² is independently selected from the group consisting of: alkyl, aminoalkyl -CN, -NO₂, -NH₂, haloalkyl, heteroaryl, phenyl, alkyl, alkoxy, alkylthio, halo and alkyl-carbonyloxy.

4. The composition of claim 3 wherein R¹ is hydroxyl or an amino group and R² is alkyl or aminoalkyl.

5. The composition of claim 3 wherein the compound is selected from the group consisting of etidronate, tiludronate, clodronate, pamidronate, alendronate, risedronate and ibandronate.

6. The composition of claim 1 further conjugated to a target tissue specific ligand.

7. The composition of claim 1 further conjugated to an imaging agent.

8. Use of a composition as claimed in any one of claims 1 to 7 in the manufacture of a medicament for destroying or impairing target cells involved in disease of bone tissue in a mammalian subject by a method comprising:
administering to the subject a therapeutically effective amount of the composition of any one of claims I to 7, wherein said composition selectively binds the target cells or target tissues involved in the disease of bone tissue; and
irradiating at least a portion of the subject with light at a wavelength or waveband absorbed by said composition, wherein said light is provided by a light source, and wherein said irradiation is at a relatively low fluence rate that results in the activation of said composition; and
wherein said composition is cleared from non-target tissues of the subject prior to said irradiation.

9. The use of claim 8, wherein said disease of bone tissue is a metabolic bone disorder or bone metastases.

10. The use of claim 8 wherein said composition is conjugated to an imaging agent.

11. The use of claim 10 in which the method further comprises the steps of performing a nuclear medicine scan and imaging the target cells or target tissues to be destroyed or impaired.

12. The use of claim 8, wherein said composition is conjugated to a ligand that specifically binds to target cells or target tissues.

13. Use of a compound which is a bisphosphonate, pyrophosphonate, thiobisphosphonate or nitrobisphosphonate in the manufacture of a medicament for destroying or impairing target cells involved in disease of bone tissue in a mammalian subject by a method comprising:
administering to the subject a therapeutically effective amount of a composition comprising a photosensitizer agent conjugated to a compound which is a bisphosphonate; pyrophosphonate; thiobisphosphonate; or nitrobisphosphonate, wherein said composition selectively binds the target cells or target tissues involved in said disease of bone tissue; and
irradiating at least a portion of the subject with light at a wavelength absorbed by said composition, wherein said light is provided by a light source, and wherein said irradiation is at a relatively low fluence rate that results in the activation of said composition, wherein said composition is cleared from non-target tissues of the subject prior to said irradiation.

14. The use of claim 13, wherein said disease of bone is a metabolic bone disorder or bone metastases.

15. Use of a compound which is a bisphosphonate; pyrophosphonate; thiobisphosphonate; or nitrobisphosphonate which selectively binds the target tissues or cells involved in a metabolic bone disorder or bone metastases in the manufacture of a medicament for treating the metabolic bone disorder or bone metastases in a mammalian subject by a method comprising:
administering to the subject a therapeutically effective amount of a composition comprising
a photosensitizer agent selected from the group consisting of chlorins, bacteriochlorins, phthalocyanines, porphyrins, purpurins, merocyanines, psoralens, benzoporphyrin derivatives (BPD), porfimer sodium, delta-aminolevulinic acid, protoporphyrin, indocyanine green (ICG), methylene blue, toluidine blue, texaphyrins and any other agent that absorbs light in a range of 500 nm -1100 nm which is conjugated to said compound and said composition is further conjugated to an imaging agent; and
performing a nuclear medicine scan;
imaging the target tissues or cells to be treated; and
irradiating at least a portion of the subject with light at a wavelength absorbed by said composition, wherein said light is provided by a light source, and wherein said irradiation is at a relatively low fluence rate that results in the activation of said composition, wherein said composition is cleared from non-target tissues of the subject prior to said irradiation.

16. Use according to claim 13 or 15, wherein said compound is a bisphosphonate of the formula 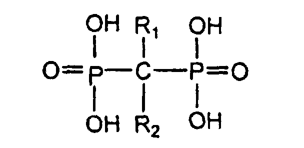 wherein R¹ is independently selected from the group consisting of: hydroxyl, an amino group, -CN, -NO₂, haloalkyl, heteroaryl, phenyl, alkyl, alkoxy, alkylthio, halo and alkyl-carbonyloxy; and wherein R² is independently selected from the group consisting of: alkyl, aminoalkyl -CN, -NO₂, -NH₂, haloalkyl, heteroaryl, phenyl, alkyl, alkoxy, alkylthio, halo and alkyl-carbonyloxy.

17. The use according to claim 16, wherein R¹ is hydroxyl or an amino group and R² is alkyl or aminoalkyl.

18. A use according to claim 13 or 15, wherein the compound is selected from the group consisting of etidronate, tiludronate, clodronate, pamidronate, alendronate, risedronate and ibandronate.

19. A use according to claim 13 or 15, wherein the composition is conjugated to a target tissue specific ligand or an imaging agent.

20. Use of a compound which is a bisphosphonate, pyrophosphonate, thiobisphosphonate or nitrobisphosphonate in the manufacture of a medicament for destroying or impairing target cells involved in disease of bone tissue in a mammalian subject by a method comprising:
administering to the subject a therapeutically effective amount of a composition comprising a photosensitizer agent, wherein said agent is selected from the group consisting of chlorins, bacteriochlorins, phthalocyanines, porphyrins, purpurins, merocyanines, psoralens, benzoporphyrin derivatives (BPD), porfimer sodium, delta-aminolevulinic acid, protoporphyrin, indocyanine green (ICG), methylene blue, toluidine blue, texaphyrins and any other agent that absorbs light in a range of 600 nm -1100 nm, and wherein said agent is conjugated to said compound which is a bisphosphonate; pyrophosphonate; thiobisphosphonate; or nitrobisphosphonate; and wherein said composition selectively binds the target cells or target tissues involved in said disease of bone tissue; and
irradiating at least a portion of the subject with light at a wavelength absorbed by said composition, wherein said light is provided by a light source, and wherein said irradiation is at a relatively low fluence rate that results in the activation of said composition; and
wherein said composition is cleared from non-target cells or non-target tissues the subject prior to said irradiation.

21. The use of claim 20, wherein said disease of bone tissue is a metabolic bone disorder or bone metastases.

22. The use of any one of claims 8-21, wherein said fluence rate results in the irradiating of said subject with a total fluence of irradiation delivered either internally or from an external light source at a range of about between 30 Joules/cm² to 25,000 Joules/cm².

23. The use of claim 22, wherein said range is between 100 Joules/cm² to 20,000 Joules/cm².

24. The use of claim 23, wherein said range is between 500 Joules/cm² to 10,000 Joules/cm².

## Patentansprüche

1. Pharmazeutische Zusammensetzung umfassend ein Fotosensibilisator-Mittel, das an eine Verbindung konjugiert ist, die ausgewählt ist aus der Gruppe bestehend aus Bisphosphonaten, Pyrophosphonaten, Thiobisphosphonaten; und Nitrobisphosphonaten.

2. Zusammensetzung nach Anspruch 1, wobei das Fotosensibilisator-Mittel aus der Gruppe ausgewählt ist bestehend aus Chlorinen, Bacteriochlorinen, Phthalocyaninen, Porphyrinen, Purpurinen, Merocyaninen, Psoralenen, Benzoporphyrin-Derivaten (BPD), Porfimer-Natrium, delta-Aminolävulinsäure, Protoporphyrin, Indocyaningrün (ICG), Methylenblau, Toluidinblau, Texaphyrinen und jedem anderen Mittel, das Licht in einem Bereich von 500 nm-1100 nm absorbiert.

3. Zusammensetzung nach Anspruch 1, wobei die Verbindung ein Bisphosphonat der Formel 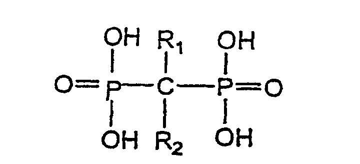 ist, worin R¹ unabhängig ausgewählt ist aus der Gruppe bestehend aus: Hydroxyl, einer Aminogruppe, -CN, -NO₂, Haloalkyl, Heteroaryl, Phenyl, Alkyl, Alkoxy, Alkylthio, Halogen und Alkylcarbonyloxy; und worin R² unabhängig ausgewählt ist aus der Gruppe bestehend aus: Alkyl, Aminoalkyl, -CN, -NO₂, NH₂, Haloalkyl, Heteroaryl, Phenyl, Alkyl, Alkoxy, Alkylthio, Halogen und Alkylcarbonyloxy.

4. Zusammensetzung nach Anspruch 3, worin R¹ Hydroxyl oder eine Aminogruppe darstellt und R² Alkyl oder Aminoalkyl darstellt.

5. Zusammensetzung nach Anspruch 3, wobei die Verbindung ausgewählt ist aus der Gruppe bestehend aus Etidronat, Tiludronat, Clodronat, Pamidronat, Alendronat, Risedronat und Ibandronat.

6. Zusammensetzung nach Anspruch 1, die ferner an einen Targetgewebe-spezifischen Liganden konjugiert ist.

7. Zusammensetzung nach Anspruch 1, die ferner an ein bildgebendes Mittel konjugiert ist.

8. Verwendung einer Zusammensetzung nach einem der Ansprüche 1 bis 7 bei der Herstellung eines Medikaments zur Zerstörung oder Beeinträchtigung von Targetzellen, die an einer Knochengewebserkrankung in einem Säuger-Patienten beteiligt sind, durch ein Verfahren, umfassend:
Verabreichung einer therapeutisch wirksamen Menge der Zusammensetzung nach einem der Ansprüche 1 bis 7 an den Patienten, wobei die Zusammensetzung die Targetzellen oder Targetgewebe, die an der Knochengewebserkrankung beteiligt sind, selektiv bindet; und
Bestrahlen zumindest eines Teils des Patienten mit Licht bei einer Wellenlänge oder in einem Wellenlängenbereich, welche/r von der Zusammensetzung absorbiert wird, wobei das Licht durch eine Lichtquelle bereitgestellt wird, und wobei die Bestrahlung eine relativ niedrige Fluenzrate aufweist, die zur Aktivierung der Zusammensetzung führt; und
wobei nicht-Targetgewebe des Patienten vor der Bestrahlung von der Zusammensetzung geklärt ist.

9. Verwendung nach Anspruch 8, wobei es sich bei der Knochengewebserkrankung um eine metabolische Knochenstörung oder Knochenmetastasen handelt.

10. Verwendung nach Anspruch 8, wobei die Zusammensetzung an ein bildgebendes Mittel konjugiert ist.

11. Verwendung nach Anspruch 10, wobei das Verfahren ferner die Schritte der Durchführung eines Nuklearmedizinscans und des Abbildens der zu zerstörenden oder zu beeinträchtigenden Targetzellen oder Targetgewebe umfasst.

12. Verwendung nach Anspruch 8, wobei die Zusammensetzung an einen Liganden konjugiert ist, der spezifisch an Targetzellen oder Targetgewebe bindet.

13. Verwendung einer Verbindung, die ein Bisphosphonat, Pyrophosphonat, Thiobisphosphonat oder Nitrobisphosphonat ist, bei der Herstellung eines Medikaments zur Zerstörung oder Beeinträchtigung von Targetzellen, die an einer Knochengewebserkrankung in einem Säuger-Patienten beteiligt sind, durch ein Verfahren, umfassend:
Verabreichung einer therapeutisch wirksamen Menge einer Zusammensetzung, umfassend ein Photosensibilisator-Mittel, das an eine Verbindung konjugiert ist, die ein Bisphosphonat, Pyrophosphonat, Thiobisphosphonat oder Nitrobisphosphonat ist, an den Patienten, wobei die Zusammensetzung die Targetzellen oder Targetgewebe, die an der Knochengewebserkrankung beteiligt sind, selektiv bindet; und
Bestrahlen zumindest eines Teils des Patienten mit Licht bei einer Wellenlänge, welche von der Zusammensetzung absorbiert wird, wobei das Licht durch eine Lichtquelle bereitgestellt wird, und wobei die Bestrahlung eine relativ niedrige Fluenzrate aufweist, die zur Aktivierung der Zusammensetzung führt;
wobei nicht-Targetgewebe des Patienten vor der Bestrahlung von der Zusammensetzung geklärt ist.

14. Verwendung nach Anspruch 13, wobei es sich bei der Knochenerkrankung um eine metabolische Knochenstörung oder Knochenmetastasen handelt.

15. Verwendung einer Verbindung, die ein Bisphosphonat, Pyrophosphonat, Thiobisphosphonat oder Nitrobisphosphonat ist, welche selektiv Targetgewebe oder Zellen bindet, die an einer metabolischen Knochenstörung oder an Knochenmetastasen beteiligt sind, bei der Herstellung eines Medikaments zur Behandlung der metabolischen Knochenstörung oder der Knochenmetastasen iri einem Säuger-Patienten, durch ein Verfahren, umfassend:
Verabreichung einer therapeutisch wirksamen Menge einer Zusammensetzung an den Patienten, umfassend
ein Fotosensibiiisator-Mittel, das ausgewählt ist aus der Gruppe bestehend aus Chlorinen, Bacteriochlorinen, Phthalocyaninen, Porphyrinen, Purpurinen, Merocyaninen, Psoralenen, Benzoporphyrin-Derivaten (BPD), Porfimer-Natrium, delta-Aminolävulinsäure, Protoporphyrin, Indocyaningrün (ICG), Methylenblau, Toluidinblau, Texaphyrinen und jedem anderen Mittel, das Licht in einem Bereich von 500 nm-1100 nm absorbiert, das an die Verbindung konjugiert ist, und die Zusammensetzung ist ferner an ein bildgebendes Mittel konjugiert; und
Durchführen eines Nuklearmedizinscans;
Abbilden der zu behandelnden Targetgewebe oder Zellen; und
Bestrahlen zumindest eines Teils des Patienten mit Licht bei einer Wellenlänge, welche von der Zusammensetzung absorbiert wird, wobei das Licht durch eine Lichtquelle bereitgestellt wird, und wobei die Bestrahlung eine relativ niedrige Fluenzrate aufweist, die zur Aktivierung der Zusammensetzung führt;
wobei nicht-Targetgewebe des Patienten vor der Bestrahlung von der Zusammensetzung geklärt ist.

16. Verwendung nach Anspruch 13 oder 15, wobei die Verbindung ein Bisphosponat der Formel 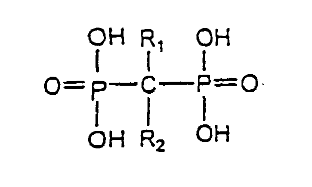 ist, worin R¹ unabhängig ausgewählt ist aus der Gruppe bestehend aus:
Hydroxyl, einer Aminogruppe, -CN, -NO₂, Haloalkyl, Heteroaryl, Phenyl, Alkyl, Alkoxy, Alkylthio, Halogen und Alkylcarbonyloxy; und worin R² unabhängig ausgewählt ist aus der Gruppe bestehend aus: Alkyl, Aminoalkyl, -CN, -NO₂, NH₂, Haloalkyl, Heteroaryl, Phenyl, Alkyl, Alkoxy, Alkylthio, Halogen und Alkylcarbonyloxy.

17. Verwendung nach Anspruch 16, worin R¹ Hydroxyl oder eine Aminogruppe darstellt und R² Alkyl oder Aminoalkyl darstellt.

18. Verwendung nach Anspruch 13 oder 15, wobei die Verbindung ausgewählt ist aus der Gruppe bestehend aus Etidronat, Tiludronat, Clodronat, Pamidronat, Alendronat, Risedronat und Ibandronat.

19. Verwendung nach Anspruch 13 oder 15, wobei die Zusammensetzung an einen Targetgewebe-spezifischen Liganden oder ein bildgebendes Mittel konjugiert ist.

20. Verwendung einer Verbindung, die ein Bisphosphonat, Pyrophosphonat, Thiobisphosphonat oder Nitrobisphosphonat ist, bei der Herstellung eines Medikaments zur Zerstörung oder Beeinträchtigung von Targetzellen, die an einer Knochengewebserkrankung in einem Säuger-Patienten beteiligt sind, durch ein Verfahren, umfassend:
Verabreichung einer therapeutisch wirksamen Menge einer Zusammensetzung an den Patienten, umfassend ein Fotosenslbllisator-Mittel, wobei das Mittel ausgewählt ist aus der Gruppe bestehend aus Chlorinen, Bacteriochlorinen, Phthalocyaninen, Porphyrinen, Purpurinen, Merocyaninen, Psoralenen, Benzoporphyrin-Derivaten (BPD), Porfimer-Natrium, delta-Aminolävulinsäure, Protoporphyrin, Indocyaningrün (ICG), Methylenblau, Toluidinblau, Texaphyrinen und jedem anderen Mittel, das Licht in einem Bereich von 600 nm-1100 nm absorbiert, und wobei das Mittel an die Verbindung konjugiert ist, die ein Bisphosphonat, Pyrophosphonat, Thiobisphosphonat oder Nitrobisphosphonat ist; und wobei die Zusammensetzung die Targetzellen oder Targetgewebe, die an der Knochengewebserkrankung beteiligt sind, selektiv bindet; und
Bestrahlen zumindest eines Teils des Patienten mit Licht bei einer Wellenlänge, welche von der Zusammensetzung absorbiert wird, wobei das Licht durch eine Lichtquelle bereitgestellt wird, und wobei die Bestrahlung eine relativ niedrige Fluenzrate aufweist, die zur Aktivierung der Zusammensetzung führt; und
wobei nicht-Targetzellen oder nicht-Targetgewebe des Patienten vor der Bestrahlung von der Zusammensetzung geklärt sind.

21. Verwendung nach Anspruch 20, wobei es sich bei der Knochengewebserkrankung um eine metabolische Knochenstörung oder Knochenmetastasen handelt.

22. Verwendung nach einem der Ansprüche 8-21, wobei die Fluenzrate zu einer Bestrahlung des. Patienten mit einer Gesamtfluenz der Bestrahlung, die entweder innerlich oder von einer externen Lichtquelle bereitgestellt wird, in einem Bereich von ungefähr zwischen 30 Joules/cm² bis 25000 Joules/cm² führt.

23. Verwendung nach Anspruch 22, wobei der Bereich zwischen 100 Joules/cm² bis 20000 Joules/cm² liegt.

24. Verwendung nach Anspruch 23, wobei der Bereich zwischen 500 Joules/cm² bis 10000 Joules/cm² liegt.

## Revendications

1. Composition pharmaceutique comprenant un agent photosensibilisant conjugué à un composé choisi dans le groupe consistant en : des bisphosphonates, des pyrophosphonates, des thiobisphosphonates et des nitrobisphosphonates.

2. Composition suivant la revendication 1, dans laquelle l'agent photosensibilisant est choisi dans le groupe consistant en des chlorines, des bactériochlorines, des phtalocyanines, des porphyrines, des purpurines, des mérocyanines, des psoralènes, des dérivés de benzoporphyrine (BPD), le porfimère sodique, l'acide delta-aminolévulinique, la protoporphyrine, le vert d'indocyanine (ICG), le bleu de méthylène, le bleu de toluidine, des texaphyrines et n'importe quel autre agent qui absorbe la lumière dans la plage de 500 nm à 1100 nm.

3. Composition suivant la revendication 1, dans laquelle le composé est un bisphosphonate de formule 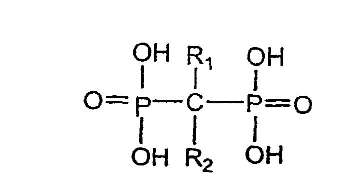 dans laquelle R¹ est choisi, indépendamment, dans le groupe consistant en : un groupe hydroxyle, un groupe amino, des groupes -CN, -NO₂, halogénalkyle, hétéroaryle, phényle, alkyle, alkoxy, alkylthio, halogéno et alkylcarbonyloxy ; et dans laquelle R² est choisi, indépendamment, dans le groupe consistant en des groupes : alkyle, aminoalkyle, -CN, -NO₂, -NH₂, halogénalkyle, hétéroaryle, phényle, alkyle, alkoxy, alkylthio, halogéno et alkylcarbonyloxy.

4. Composition suivant la revendication 3, dans laquelle R¹ représente un groupe hydroxyle ou un groupe amino et R² représente un groupe alkyle ou aminoalkyle.

5. Composition suivant la revendication 3, dans laquelle le composé est choisi dans le groupe consistant en l'étidronate, le tiludronate, le clodronate, le pamidronate, l'alendronate, le risédronate et l'ibandronate.

6. Composition suivant la revendication 1, conjuguée en outre à un ligand spécifique d'un tissu cible.

7. Composition suivant la revendication 1, conjuguée en outre à un agent d'imagerie.

8. Utilisation d'une composition suivant l'une quelconque des revendications 1 à 7 dans la production d'un médicament destiné à détruire ou entraver des cellules cibles impliquées dans une maladie du tissu osseux chez un sujet consistant en un mammifère, par une méthode comprenant les étapes consistant :
à administrer au sujet une quantité thérapeutiquement efficace de la composition suivant l'une quelconque des revendications 1 à 7, ladite composition se liant sélectivement aux cellules cibles ou tissus cibles impliqués dans la maladie du tissu osseux ; et
à irradier au moins une partie du sujet avec de la lumière à une longueur d'onde ou bande d'ondes absorbée par ladite composition, ladite lumière étant fournie par une source de lumière, et ladite irradiation étant à une vitesse de fluence relativement faible ayant pour résultat l'activation de ladite composition ; et
ladite composition étant éliminée des tissus non cibles du sujet avant ladite irradiation.

9. Utilisation suivant la revendication 8, dans laquelle ladite maladie du tissu osseux est un trouble osseux métabolique ou des métastases osseuses.

10. Utilisation suivant la revendication 8, dans laquelle ladite composition est conjuguée à un agent d'imagerie.

11. Utilisation suivant la revendication 10, dans laquelle la méthode comprend en outre les étapes consistant à effectuer un balayage médical nucléaire et une imagerie des cellules cibles ou tissus cibles à détruire ou entraver.

12. Utilisation suivant la revendication 8, dans laquelle ladite composition est conjuguée à un ligand qui se lie spécifiquement à des cellules cibles ou tissus cibles.

13. Utilisation d'un composé qui est un bisphosphonate, un pyrophosphonate, un thiobisphosphonate ou un nitrobisphosphonate dans la production d'un médicament destiné à détruire ou entraver des cellules cibles impliquées dans une maladie du tissu osseux chez un sujet consistant en un mammifère, par une méthode comprenant les étapes consistant :
à administrer au sujet une quantité thérapeutiquement efficace d'une composition comprenant un agent photosensibilisant conjugué à un composé qui est un bisphosphonate, un pyrophosphonate, un thiobisphosphonate ou un nitrobisphosphonate, ladite composition se liant sélectivement aux cellules cibles ou tissus cibles impliqués dans ladite maladie du tissu osseux ; et
à irradier au moins une partie du sujet avec de la lumière à une longueur d'onde absorbée par ladite composition, ladite lumière étant fournie par une source lumineuse, et ladite irradiation étant effectuée à une vitesse de fluence relativement faible ayant pour résultat l'activation de ladite composition, ladite composition étant éliminée des tissus non cibles du sujet avant ladite irradiation.

14. Utilisation suivant la revendication 13, dans laquelle ladite maladie du tissu osseux est un trouble osseux métabolique ou des métastases osseuses.

15. Utilisation d'un composé qui est un bisphosphonate, un pyrophosphonate, un thiobisphosphonate ou un nitrobisphosphonate qui se lie sélectivement aux tissus cibles ou cellules cibles impliqués dans un trouble osseux métabolique ou des métastases osseuses, dans la production d'un médicament destiné au traitement du trouble osseux métabolique ou des métastases osseuses chez un sujet consistant en un mammifère, par une méthode comprenant les étapes consistant :
à administrer au sujet une quantité thérapeutiquement efficace d'une composition comprenant
un agent photosensibilisant choisi dans le groupe consistant en des chlorines, des bactériochlorines, des phtalocyanines, des porphyrines, des purpurines, des mérocyanines, des psoralènes, des dérivés de benzoporphyrine (BPD), le porfimère sodique, l'acide delta-aminolévulinique, la protoporphyrine, le vert d'indocyanine (ICG), le bleu de méthylène, le bleu de toluidine, des texaphyrines et n'importe quel autre agent qui absorbe la lumière dans la plage de 500 nm à 1100 nm, qui est conjugué audit composé, et ladite composition est en outre conjuguée à un agent d'imagerie ; et
à effectuer un balayage médical nucléaire ;
à effectuer l'imagerie des tissus cibles ou cellules cibles à traiter ; et
à irradier au moins une partie du sujet avec de la lumière à une longueur d'onde absorbée par ladite composition, ladite lumière étant fournie par une source de lumière, et ladite irradiation étant effectuée à une vitesse de fluence relativement faible ayant pour résultat l'activation de ladite composition, ladite composition étant éliminée des tissus non cibles du sujet avant ladite irradiation.

16. Utilisation suivant la revendication 13 ou 15, dans laquelle ledit composé est un bisphosphonate de formule 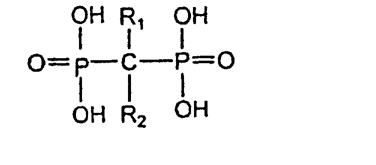 dans laquelle R¹ est choisi, indépendamment, dans le groupe consistant en : un groupe hydroxyle, un groupe amino, des groupes -CN, -NO₂, halogénalkyle, hétéroaryle, phényle, alkyle, alkoxy, alkylthio, halogéno et alkylcarbonyloxy ; et dans laquelle R² est choisi, indépendamment, dans le groupe consistant en des groupes : alkyle, aminoalkyle, -CN, -NO₂, -NH₂, halogénalkyle, hétéroaryle, phényle, alkyle, alkoxy, alkylthio, halogéno et alkylcarbonyloxy.

17. Utilisation suivant la revendication 16, dans laquelle R¹ représente un groupe hydroxyle ou un groupe amino et R² représente un groupe alkyle ou aminoalkyle.

18. Utilisation suivant la revendication 13 ou 15, dans laquelle le composé est choisi dans le groupe consistant en l'étidronate, le tiludronate, le clodronate, le pamidronaté, l'alendronate, le risédronate et l'ibandronate.

19. Utilisation suivant la revendication 13 ou 15, dans laquelle la composition est conjuguée à un ligand spécifique du tissu cible ou un agent d'imagerie.

20. Utilisation d'un composé qui est un bisphosphonate, un pyrophosphonate, un thiobisphosphonate ou un nitrobisphosphonate dans la production d'un médicament destiné à détruire ou entraver des cellules cibles impliquées dans une maladie du tissu osseux chez un sujet consistant en un mammifère, par une méthode comprenant les étapes consistant :
à administrer au sujet une quantité thérapeutiquement efficace d'une composition comprenant un agent photosensibilisant, ledit agent étant choisi dans le groupe consistant en des chlorines, des bactériochlorines, des phtalocyanines, des porphyrines, des purpurines, des mérocyanines, des psoralènes, des dérivés de benzoporphyrine (BPD), le porfimère sodique, l'acide delta-aminolévulinique, la protoporphyrine, le vert d'indocyanine (ICG), le bleu de méthylène, le bleu de toluidine, des texaphyrines et n'importe quel autre agent qui absorbe la lumière dans la plage de 600 nm à 1100 nm, ledit agent étant conjugué audit composé qui est un bisphosphonate, un pyrophosphonate, un thiobisphosphonate ou un nitrobisphosphonate ; ladite composition se liant sélectivement aux cellules cibles ou tissus cibles impliqués dans ladite maladie du tissu osseux ; et
à irradier au moins une partie du sujet avec de la lumière à une longueur d'onde absorbée par ladite composition, ladite lumière étant fournie par une source de lumière, et ladite irradiation étant effectuée à une vitesse de fluence relativement faible ayant pour résultat l'activation de ladite composition ; et
ladite composition étant éliminée des cellules non cibles ou tissus non cibles du sujet avant ladite irradiation.

21. Utilisation suivant la revendication 20, dans laquelle ladite maladie du tissu osseux est un trouble osseux métabolique ou des métastases osseuses.

22. Utilisation suivant l'une quelconque des revendications 8 à 21, clans laquelle ladite vitesse de fluence a pour résultat l'irradiation dudit sujet à une fluence totale d'irradiation délivrée intérieurement ou par une source de lumière extérieure dans l'intervalle d'environ 30 joules/cm² à 25 000 joules/cm².

23. Utilisation suivant la revendication 22, dans laquelle ledit intervalle va de 100 joules/cm² à 20 000 joules/cm².

24. Utilisation suivant la revendication 23, dans laquelle ledit intervalle va de 500 joules/cm² à 10 000 joules/cm².
